(19) Europäisches Patentamt
European Patent Office
Office européen des brevets

(11) Publication number: 0 304 208 B1

# (12) EUROPEAN PATENT SPECIFICATION

(45) Date of publication of patent specification: 12.05.93 (51) Int. Cl.5: C25B 3/02, //C07D205/08

(21) Application number: 88307324.9

(22) Date of filing: 08.08.88

## (54) Chemical process.

(30) Priority: 20.08.87 GB 8719695

(43) Date of publication of application:
22.02.89 Bulletin 89/08

(45) Publication of the grant of the patent:
12.05.93 Bulletin 93/19

(84) Designated Contracting States:
AT BE CH DE ES FR GB GR IT LI LU NL SE

(56) References cited:

CHEMICAL ABSTRACTS, vol. 103, No. 3, July 22, 1985, Columbus, Ohio, USA OKITA, MAKOTO et al.: "Anodic oxidation of N-alkyl-beta-lactams." page 558, abstract No. 22 389f & HETEROCYCLES 1985, 23(2), 247-50

CHEMICAL ABSTRACTS, vol. 103, No. 3, July 22, 1985, Columbus, Ohio, USA MORI, MIWAKO et al. "Debenzylation of N-benuzyl-beta-lactams by use of anodic oxidation." page 558, abstract No. 22 390z & HETEROCYCLES 1985, 23(2), 317-23

(73) Proprietor: IMPERIAL CHEMICAL INDUSTRIES PLC
Imperial Chemical House, Millbank
London SW1P 3JF(GB)

(72) Inventor: Oldham, Keith
9 Lambourn Close
Poynton Cheshire SK12 1UG(GB)
Inventor: Betts, Michael John
7 Beechway
Wilmslow Cheshire SK9 6LB(GB)

(74) Representative: Denerley, Paul Millington, Dr. et al
ICI Group Patents Services Dept. PO Box 6
Shire Park Bessemer Road
Welwyn Garden City Herts, AL7 1HD (GB)

EP 0 304 208 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process of removing protecting groups and in particular to a process of removing certain phenyl protecting groups from the ring nitrogen atom of azetidinones.

Protecting groups are of general utility in organic chemistry as they prevent, or minimise, chemical reactions at certain positions of a molecule. Thus they enable the desired transformation or transformations to proceed elsewhere in the molecule. In general protecting groups should satisfy three criteria; they should be readily introduced into the molecule, they should be substantially stable during the desired transformation and they should be readily removable without causing significant degradation of the remainder of the molecule. That is they should be introduced and removed in high yields. This is particularly important for the commercialisation of organic syntheses where it is particularly beneficial to have high, economic yields and to avoid the need for unnecessary and/or elaborate purification of compounds.

The above is particularly true in β-lactam (azetidinone) chemistry. In the past few years many groups have been investigating the synthesis, from acyclic precursors, of carbapenems, penems, monobactams, and penicillin and cephalosporin analogues and derivatives. These syntheses have relied heavily on the ability to use protecting groups. However it is acknowledged that the β-lactam ring is susceptible to irreversible cleavage under many conditions and therefore many protecting groups are not suitable because degradation occurs when it is attempted to remove them. Furthermore many protecting groups are only removable to give the desired product in low yield, with the need for expensive and complicated purification, because of the relative instability of the β-lactam ring under the conditions necessary for removal of the protecting group.

A particular class of protecting group favoured for the nitrogen atom of a monocyclic β-lactam:-

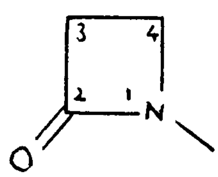

is typified by the 4-methoxyphenyl group. This is readily incorporated into intermediates prior to formation of the β-lactam ring. Furthermore it is substantially stable whilst transformations are effected on the substituents at positions 3 and 4 of the ring. However, unfortunately, the yields for processes in which such a group is removed can be low and uneconomic. EP-A-181831 exemplifies the use of this N-protecting group. It is stated to be removable by ozonolysis and subsequent decomposition of the ozonides by reduction with a reducing agent, or by reaction with a cerium (IV) salt for example diammonium cerium (IV) hexanitrate. In the examples using cerium (IV) ammonium nitrate, no yields are given but large excesses of cerium reagent are used rendering purification relatively difficult. We have found that this deprotection method when performed on similar compounds can result in low yields and a mixture of by-products. Some improvement can be made by varying the reaction conditions but in general the process is relatively unsatisfactory for commercialisation. Clearly the large excess of reagent, the disposal problems associated with the heavy metal, the potential explosive hazard of cerium in organic solvents, the necessity for difficult chromatography and the low yields are problems that need to be overcome in order to improve the economics of any syntheses that process through these β-lactam intermediates.

The present invention provides a solution to these problems of the art by removing such N-protecting groups by electrochemical oxidation. This method affords higher yields with an improved impurity profile. It significantly reduces the difficulty of purification. Furthermore it avoids the need for expensive reagents and it avoids the disposal problems associated with the heavy metal. In addition the ceric ammonium nitrate deprotection is conducted at high acidity which means that acid-labile protecting groups are not so suitable for the azetidinone thereby limiting the synthetic possibilities.

Accordingly the present invention provides a process for preparing an azetindin-2-one wherein the ring nitrogen atom carries a hydrogen atom which process comprises electrochemically oxidising an azetidin-2-one wherein the ring nitrogen atom is substituted by a phenyl group carrying an ortho- or para-substituent selected from hydroxy, optionally substituted alkoxy, optionally substituted phenoxy, alkenyloxy, protected hydroxy, amino, $C_{1-6}$alkylamino, di-$C_{1-6}$alkylamino, arylamino, di-arylamino, acylamino or di-acylamino, in a liquid medium which comprises water and optionally an organic solvent substantially inert to electrolytic oxidation, in the presence of a working electrode at a potential effective to

oxidise and subsequently remove the N-substituent without undesired substantial oxidation of the remainder of the molecule.

In a particular aspect the compounds prepared by the process of the present invention are those of the formula I: -

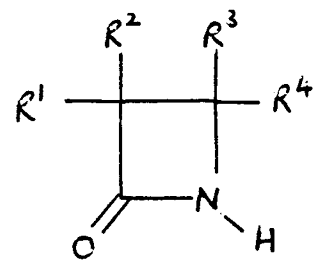

wherein $R^1-R^4$ are independently hydrogen, halogen or an organic group or $R^1$ and $R^2$ are joined together to form a spiro compound It will be appreciated by the skilled man that the exact nature of the groups $R^1-R^4$ is not critical to the process of this invention provided that the groups $R^1-R^4$ are substantially stable during the electrochemical oxidation, unless of course it is desired to effect another transformation concurrently with the N-deprotection.

More particularly $R^1$ and $R^2$ are independently hydrogen, optionally substituted $C_{1-6}$ alkyl, optionally substituted $C_{2-6}$alkenyl, optionally substituted $C_{2-6}$alkynyl, $C_{3-8}$cycloalkyl, aryl, optionally substituted $C_{1-6}$alkoxy, halo, optionally substituted $C_{2-6}$alkenyloxy, azido, acylamino, hydroxy, $C_{1-6}$alkanoyloxy, $C_{1-6}$alkanoyl, arylcarbonyl, arylcarbonyloxy, $C_{1-6}$alkylthio, $C_{1-6}$alkyldithio, arylthio, carboxy, $C_{1-6}$alkoxycarbonyl,aryloxycarbonyl and the like wherein aryl is optionally substituted phenyl; and optional substituents include $C_{1-6}$alkyl, $C_{2-6}$alkenyl, phenyl, heteroaryl or heterocyclyl wherein the heteroatoms are selected from 1-4 oxygen, sulphur or nitrogen atoms, hydroxy, $C_{1-6}$alkoxy, $C_{1-6}$alkylamino, di-$C_{1-6}$alkylamino, $C_{1-6}$alkylthio, sulphamoyl, ureido, amidino, guanidino, bromo, chloro, fluoro, cyano, carboxy, nitro and the like; any functional group being protected by a protecting group if desired.

Preferably $R^1$ is $C_{1-6}$alkyl for example methyl, ethyl or propyl, hydroxy $C_{1-6}$alkyl for example hydroxymethyl, 1-hydroxyethyl or 2-hydroxyprop-2-yl, protected hydroxy $C_{1-6}$alkyl, azido, protected amino, acylamino for example the C-6 side chains of the penicillin art, the C-7 side chains of the cephalosporin art and the C-3 side chains of the monobactam art, $C_{1-6}$alkoxy for example methoxy, halo for example bromo, chloro or iodo, halo$C_{1-6}$alkyl for example 1-fluoroethyl or hydrogen.

Preferably $R^2$ is hydrogen, halo for example boro or when $R^1$ is protected amino or acylamino $R^2$ is also conveniently methoxy or formamido.

More particularly $R^3$ and $R^4$ are independently hydrogen, optionally substituted $C_{1-6}$ alkyl, optionally substituted $C_{2-6}$alkenyl, optionally substituted $C_{2-6}$alkynyl, $C_{3-8}$cycloalkyl, aryl, optionally substituted $C_{1-6}$alkoxy, halo, optionally substituted $C_{2-6}$alkenyloxy, azido, acylamino, di-$C_{1-6}$alkoxyphosphinyl, $C_{1-6}$-alkanoyloxy, $C_{1-6}$alkanoyl, arylcarbonyl, arylcarbonyloxy, $C_{1-6}$alkylthio, $C_{1-6}$alkyldithio, arylthio, carboxy, $C_{1-6}$alkoxycarbonyl,$C_{1-6}$alkanoylthio, arylcarbonylthio, aryloxycarbonyl and the like wherein aryl is optionally substituted phenyl; and optional substituents include $C_{1-6}$alkyl, $C_{2-6}$alkenyl, phenyl, heteroaryl or heterocyclyl wherein the heteroatoms are selected from 1-4 oxygen, sulphur or nitrogen atoms, hydroxy, $C_{1-6}$alkoxy, $C_{1-6}$alkylamino, di-$C_{1-6}$alkylamino, $C_{1-6}$alkylthio, sulphamoyl, ureido, amidino, guanidino, bromo, chloro fluoro, cyano, carboxy, nitro and the like; any functional group being protected by a protecting group if desired.

Preferably $R^3$ is hydrogen.

Preferably $R^4$ is carboxy, $C_{1-6}$alkoxycarbonyl for example methoxycarbonyl, ethoxycarbonyl or t-butoxycarbonyl, $C_{1-6}$alkanoyl for example acetyl, propionyl or pivaloyl, arylcarbonyl for example benzoyl, p-chlorobenzoyl or p-nitrobenzoyl, arylcarbonyloxy for example benzoyloxy, p-chlorobenzoyloxy or p-nitrobenzoyloxy, $C_{1-6}$-alkanoyloxy for example acetoxy, propionoxy or pivaloyloxy, $C_{1-6}$-alkylthio for example methylthio, $C_{1-6}$alkyldithio for example $CH_3S-S-$, $C_{1-6}$alkanoylthio for example acetylthio, di-$C_{1-6}$alkoxyphosphinyl for example diethoxyphosphinyl, or azido.

Where the nature of the substituents allows the process of the present invention covers the preparation of both optically active and racemic compounds as well as both cis and trans azetidinones and mixtures thereof.

As stated previously the azetidinone that is electrochemically oxidised is N-substituted by certain groups.

In a particular aspect this N-substituent is of the formula II:

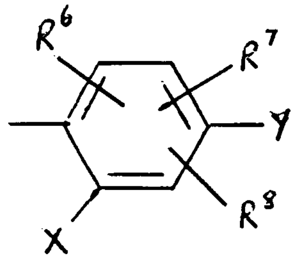

wherein one of X and Y is a group $-OR^5$ where $R^5$ is hydrogen, a hydroxy protecting group, optionally substituted $C_{1-6}$alkyl, optionally substituted phenyl or $C_{2-6}$alkenyl, the other of X and Y is a group $R^9$ and $R^6-R^9$ are selected from hydrogen, hydroxy, protected hydroxy, optionally substituted $C_{1-6}$alkyl, optionally substituted $C_{1-6}$alkoxy, optionally substituted phenoxy, $C_{2-6}$alkenyloxy, halo, nitro $C_{1-6}$-alkanoylamino, aryl and heteroaryl.

Preferably Y is $C_{1-6}$alkoxy for example methoxy and X, $R^6$, $R^7$ and $R^8$ are all hydrogen, that is the 4-methoxyphenyl group.

In another particular aspect the N-substituent is of the formula III:

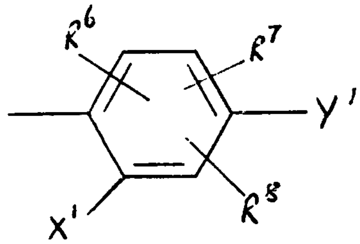

wherein one of X' and Y' is a group $-NR^{10}R^{11}$ wherein $R^{10}$ and $R^{11}$ are independently hydrogen, $C_{1-6}$alkyl for example methyl, aryl for example phenyl or acyl for example $C_{1-6}$alkanoyl such as acetyl, the other of X' and Y' is a group $R^9$ and $R^6-R^9$ are as defined above.

When reference is made to protecting groups being present at any position in the compounds described herein such protecting groups may in general be chosen from any of the groups described in the literature or known to the skilled chemist as appropriate for the protection of the group in question, and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule. Of course they should not be removable by the conditions of the electrochemical process of the present invention, unless it is so desired.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxyl protecting group may be the residue of an ester-forming aliphatic or araliphatic alcohol or of an ester-forming phenol or silanol (the said alcohol, phenol or silanol preferably containing 1-20 carbon atoms).

Examples of carboxy protecting groups include straight or branched chain (1-12C)alkyl groups (eg isopropyl, t-butyl); halo lower alkyl groups (eg 2-chloroethyl, 2,2,2-trichloroethyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxy-carbonyloxyethyl, 1-ethoxycarbonyloxyethyl); aryl lower

alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); and (2-6C)alkenyl groups (eg allyl and vinylethyl).

Methods particularly appropriate for the removal of carboxyl protecting groups include for example acid-, base-, metal- or enzymically-catalysed hydrolysis.

Examples of hydroxyl protecting groups include lower alkanoyl groups (eg acetyl); allyloxycarbonyl; lower alkoxycarbonyl groups (eg t-butoxycarbonyl); halo lower alkoxycarbonyl groups (eg 2-iodoethoxycarbonyl, 2,2,2-trichloroethoxycarbonyl); aryl lower alkoxycarbonyl groups (eg benzyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl, tri-isopropylsilyl and dimethyl-1,1,2-trimethylpropylsilyl), aryloxydialkylsilyl (for example 2,6-di-t-butyl-4-methylphenoxydimethylsilyl), aryldialkylsilyl (for example diphenyl-t-butylsilyl) and aryl lower alkyl (eg benzyl) groups.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; acyl (eg allyloxycarbonyl, alkoxycarbonyl and aralkoxycarbonyl eg t-butoxycarbonyl and benzyloxycarbonyl); trialkylsilyl (eg trimethylsilyl and t-butyldimethylsilyl); alkylidene (eg methylidene); benzylidene and substituted benzylidene groups; and the phthalimido group.

The process of the present invention may be performed in any conventional type of electrolytic cell. An electrochemical reaction necessarily involves an anode, a cathode and a liquid medium. In electrochemical oxidations the anode is termed the working electrode, that is the electrode at which the reaction occurs. The cathode is termed the auxiliary electrode. The reaction can either be performed in a two-electrode cell or a three-electrode cell may be used in which case the third electrode is a reference electrode which functions only to act as a reference against which the potential of the working electrode can be measured. The potential of this working electrode is varied by altering the voltage that is applied to the cell. The greater the positive potential then the greater the oxidising power. The oxidation of this invention is a two-electron process so that a minimum of 2 Farads per mole is required for complete reaction.

The reaction may also be performed in a cell containing more than one anode and more than one cathode, that is a cell containing a number of units each of which contains an anode and a cathode.

The working electrode (anode) is formed of any material effective for the oxidation process of the present invention. Such materials include platinum, carbon including graphite, glassy carbon, silver oxide, manganese oxide, iron oxide and precious metals such as gold, ruthenium, iridium and rhodium. Usually precious metals and precious metal oxides are coated on a support for example another metal as it is the electrode surface that is of most importance in electrochemical reactions for example platinised titanium is particularly useful. The working electrode can also be formed from alloys. The physical form of the electrode is not critical and any of the art-recognised forms are suitable for example plates, rods, wires, gauzes, wools, sheets, etc.

The auxiliary electrode (cathode) can be selected from materials known in the art for example mercury, lead, tin, zinc, cadmium, nickel, chromium, aluminium, copper, iron, steel, carbon including graphite, silver, platinum, gold and alloys. The cathode may be used in any of the art-recognised forms.

The reference electrode, if present, can be any art-recognised electrode known for this purpose, for example a calomel electrode.

As stated above, the present process can be performed in any conventional electrolytic cell. One type of cell is the static cell wherein it is conventional to agitate the liquid medium so as to keep the surface of the working electrode clean and to continually present a compound to be oxidised to the electrode surface. Another type of cell is the flow cell, more suited for large-scale production, wherein the liquid medium is continually passed through the cell, the flow of liquid removing the need for agitation. Static and flow cells are well known in the art and in general are designed so that the working electrode and the auxiliary electrode are spaced by a constant, small distance apart in order to maximise current flow and minimise temperature rise due to the internal resistance of the fluid to the current flow.

It is fundamental that all electrochemical cell reactions involve at least two opposite reactions, that is a reduction and an oxidation. If the reduction, associated with the oxidation process of the present invention, does not cause substantial degradation or undesired reaction then the electrolytic cell can be an undivided cell. That is the liquid medium is uniform throughout the cell. However if the reduction process leads to undesirable degradation or reaction then the process of the present invention is performed in a divided cell. In such a cell, well-known in the art, the working electrode and the auxiliary electrode are immersed in different fluids that are physically separate but are electrically connected. The divider can be made of any material known in the art for this purpose for example ceramics, asbestos, sintered glass, natural and synthetic membranes, and ion-exchange membranes for example Nafion (Nafion is a Trade Mark of

Dupont). In addition a conducting gel, permeable to ions, may be used.

It will be appreciated that in divided cells the compound being deprotected by oxidation is situated in that part of the cell adjacent the anode. It is not able to contact the cathode so that some other balancing cell reaction must occur. This is possible through the solvent itself or through an ion of the electrolyte or other appropriate substance.

The aqueous medium in the process of the present invention comprises water and, in a preferred aspect, an organic solvent that is substantially inert to the reaction conditions is present to facilitate solubilisation of the starting material. Conveniently up to about 50% water (vol/vol) is present, for example 10% – 40%. The organic solvent may be immiscible with water or miscible with water. It is preferred to use a water – miscible solvent so as to provide a homogeneous solution of the compound to be oxidised. Suitable organic solvents include alcohols for example methanol, ethanol and propanol, acetone, acetonitrile, dimethylformamide and tetrahydrofuran. Water – immiscible solvents include halogenated hydrocarbons for example dichloromethane and chloroform, benzene, toluene, alkanes for examples pentane and hexane, ether and ketones such as methylbutyl ketone. It will be appreciated that it is necessary to provide an electrolyte to enable an electric current to be conducted whether the liquid medium is one – phase or two – phase. Suitable electrolytes for the process of the present invention, include lithium salts for example the perchlorate and tetrafluoroborate, sodium salts for example the perchlorate, sulphate and tetrafluoroborate, ammonium ($R_4N^+$) salts for example the tetrafluoroborates and perchlorates and various sulphonates such as tetramethylammonium methyl sulphate and tetraethylammonium ethyl sulphate. Further suitable electrolytes include nitrates, sulphates, phosphates and hexafluorophosphates.

It is thought that the process of the present invention proceeds via an electrochemical oxidation step to form an intermediate species that is hydrolysed in the aqueous medium to provide the azetidin – 2 – one wherein the ring nitrogen atom carries a hydrogen atom.

We have found it convenient to perform the reaction at a non – extreme pH value in order to minimise by – products, for example in the range pH2 – 12 and more preferably in the range 4 – 7. The anolyte, that is the liquid medium in the anode compartment of a divided cell, will become progressively more acidic and a pH value in the desired range can be maintained by the addition of base.

The process is performed at any convenient temperature for example between −5 ° C and 85 ° C, preferably between 10 ° C and 40 ° C and conveniently at ambient temperature.

The potential of the working electrode is controllable by methods known to those skilled in the art. Conveniently a potentiostat is used which measures the potential between the working electrode and the reference electrode and automatically alters the voltage between the working electrode and the auxiliary electrode to maintain the desired potential.

The potential at which the process of the present invention is operable will, of course, vary from cell to cell. However for any particular system a suitable potential range can be determined by methods conventional in the art.

Conveniently the potential of the system is such that the desired N – deprotection by oxidation occurs without any substantial oxidative degradation or other oxidation reacctions occurring. The preferred potential can be readily determined in standard manner for example by constructing a voltammogram curve for any particular system.

In general the potential of the working electrode for the oxidations of the present process have been determined to be in the range 1.2 – 1.8 V relative to a calomel reference electrode.

The current density is generally in the range of 1 – 100mA/$cm^2$ and can be lowered during a process to minimise the possibility of over – oxidation.

The following Examples, in which electrolyses were carried out in an H – cell (unless otherwise stated) divided by a Nafion cationic exchange resin, using platinum electrodes (4 cm2) in both anode and cathode compartments, serve to illustrate the invention. Nmr shifts are quoted in ppm (δ) downfield from tetramethylsilane. Temperature are quoted in degrees Celsius ( ° C). Hplc represents high performance liquid chromatography; mplc represents medium pressure liquid chromatography; DMSO represents dimethylsulphoxide.

## Example 1

(3S,4S,1′R) – 4 – Benzoyl – 3 – (1′ – hydroxyethyl) – 1 – (p – methoxyphenyl)azetidin – 2 – one (1 g), dissolved in acetonitrile (20 ml) and water (10 ml) containing tetrabutylammonium tetrafluoroborate (0.5 g) was placed in the anode compartment of an H – cell. The cathode compartment contained the same components apart from the azetidinone. The mixture was electrolysed for 4.8 hours at constant voltage (15 V), at ambient temperature. The pH was adjusted to 7 with solid sodium bicarbonate, extracted with ethyl acetate

(20 ml), the organic layer was washed with 10% aqueous sodium sulphite, dried, and evaporated. After chromatography (mplc) using a gradient from 90:10 petrol (bp 60 – 80°)/ethyl acetate to ethyl acetate, appropriate fractions were combined and evaporated to give (3S,4S,1'R) – 4 – benzoyl – 3 – (1' – hydroxyethyl)azetidin – 2 – one (0.35g, 52%). A sample recrystallised from methylisobutylketone had mp 131 – 133°.
Nmr (d6 – DMSO, 200MHz): 1.13 (3H, d, J = 6.1 Hz); 2.99 (1H, dt, J = 2.3 and 6.1 Hz); 4.02 (1H, hextet, J = 6.1 Hz); 5.07 (1H,d, J = 2.3Hz); 5.15, d, J = 6.1 Hz); 7.55 (2H, m); 7.69 (1H, m); 8.04 (2H, m); 8.36 (1H, brs).

## Example 2

(3S,4S,1'R) – 4 – Benzoyl – 3 – (1' – hydroxyethyl) – 1 – (p – methoxyphenyl)azetidin – 2 – one (1 g), dissolved in ethyl acetate (30 ml) was mixed with a solution of tetrabutylammonium tetrafluoroborate (0.5 g) in water (10 ml) and placed in the anode compartment of an H – cell. The cathode compartment was filled with a solution of tetrabutylammonium tetrafluoroborate (1.5 g) in water (30 ml), and electrolysis carried out for 6 hours at constant voltage (30 V) and ambient temperature. The separated organic layer was washed with sodium bicarbonate, sodium sulphite and brine, then subjected to chromatography as in Example 1 to give the same product (0.4 g, 61%).

## Examples 3– 9

Using the same general techniques and substrate as in Example 1, the following Examples were carried out. The yields are by hplc relative to a standard solution; TBAF is tetrabutylammonium tetrafluoroborate.

| Ex | Scale (g) | Solvent | Electrolyte (anode) | Product yield % | Experiment at constant | | Time (hr) | Note |
|---|---|---|---|---|---|---|---|---|
| | | | | | volts(V) | amps(mA) | | |
| 3 | 1.0 | $CH_3CN/H_2O$ | TBAF | 82 | 30 | – | 3.0 | |
| 4 | 1.0 | $CH_3CN/H_2O$ (+NaOH) | TBAF | 72 | 30 | – | 2.5 | 1 |
| 5 | 1.0 | $CH_3CN/H_2O$ (+$NaHCO_3$) | TBAF | 83 | 30 | – | 2.5 | 2 |
| 6 | 1.0 | $EtOAc/H_2O$ | $NaBF_4$ | 55 | 30 | – | 6.0 | |
| 7 | 1.0 | $CH_3CN/H_2O$ | $NaBF_4$ | 58 | 20 | – | 2.5 | 3 |
| 8 | 1.0 | $CH_3CN/H_2O$ | $NaBF_4$ | 88 | – | 100 | 2.5 | |
| 9 | 2.0 | $CH_3CN/H_2O$ (+$NaHCO_3$) | $NaBF_4$ | 92 | – | 100 | 2.5 | 4 |

Notes:
1 pH adjusted by addition of aqueous sodium hydroxide during reaction
2 pH adjusted by addition of solid sodium bicarbonate during reaction
3 Reaction carried out at ice temperature
4 Reaction carried out in presence of excess solid sodium bicarbonate

## Examples 10– 14

Using the same general technique and substrate as in Example 1 above, and on the same scale and at the same temperature, the following Examples show the effect of varying the electrolyte and the method of buffering the solution; yields are by hplc relative to a standard solution.

| Ex | Electrolyte | Solvent $CH_3CN:H_2O$ Ratio | Current (mA) | Voltage (V) | Time (hr) | Product (% by hplc) | Notes |
|---|---|---|---|---|---|---|---|
| 10 | 5% $NaH_2PO_4$ | 2:1 | 25 – 30 | 28* | 6 | 45 | 1 |
| 11 | 5% $Na_2SO_4$ | 2:1 | 107* | 15 – 30 | 5.5 | 50 | 2 |
| 12 | 2% $Na_2SO_4$ + 3% $NaH_2PO_4$ | 1:1 | 110* | ~15 | 4 | 60 | 3 |
| 13 | 5% $LiBF_4$ | 9:1 | 110* | 14 – 22 | 2 | 73 | 4 |
| 14 | 10% $Na_2HPO_4$ + 2% $NaBF_4$ | 7:3 | 97* | 13 – 20 | 2.8 | 76 | 5 |
| 15 | 3% $KNO_3$ | 7:3 | 80 – 95 | 13* | 3 | 67 | 6 |

Notes
1. pH drops to 2.
2. Solid NaHCO3 added; yield by hplc after extractive work – up as Example 1.
3. Solid NaHCO3 added. yield by hplc after extractive work – up as Example 1.
4. Solid NaHCO3 added.
5. Good pH control at 4 – 5; two phases through most of reaction.
6. pH controlled at 5.

* Indicates which of current or voltage kept constant.

**Example 16**

(3S,4S,1′R) – 4 – Benzoyl – 3 – (1′ – hydroxyethyl) – 1 – (p – methoxyphenyl)azetidin – 2 – one (1 g), dissolved in a mixture of acetonitrile (20 ml) and 5% aqueous sodium tetrafluoroborate (10 ml) was placed in the anode compartment of an H – cell. Solid sodium bicarbonate (1.5 g) was then added to the anode compartment. The cathode compartment was filled with a 5% aqueous solution of sodium tetrafluoroborate maintained at approximately pH 2 by the addition of concentrated hydrochloric acid. Electrolysis was carried out maintaining the potential of the anode at less than 1.7V by reducing the cell current. The electrode potential of the anode was measured by conventional means relative to a calomel electrode. After this process was complete the anode solution was treated with a solution of sodium sulphite (0.5 g) in water (2 ml) and allowed to stand for 10 minutes. The solution was concentrated to low volume and extracted with ethyl acetate (3 x 30 ml). The organic extracts were washed with 0.25N sodium hydroxide solution (10 ml) and saturated sodium chloride (10 ml) and then dried over magnesium sulphate. The yield was estimated by hplc at 85%. The ethyl acetate solution was evaporated to dryness to yield (3S,4S,1′R) – 4 – benzoyl – 3 – (1′ – hydroxyethyl)azetidin – 2 – one as a pale brown oil (0.70g), which crystallised on standing.

**Example 17**

(3S,4S,1′R) – 4 – Benzoyl – 3 – (1′ – hydroxyethyl) – 1 – (p – methoxyphenyl)azetidin – 2 – one (14 g), dissolved in a mixture of acetonitrile (150 ml) and 5% aqueous sodium tetrafluoroborate (75 ml) was placed in the reservoir of a flow – cell, with 5% aqueous sodium tetrafluoroborate in the cathode reservoir. The flow – cell had electrodes of platinised titanium of approximate area 30 $cm^2$. The solutions were pumped around the cell and a constant current of 800 mA applied for 7 hours. During this period the anode compartment was kept within the pH range 3 – 7 by the addition of solid sodium bicarbonate to the circulating electrolyte and the cathode compartment maintained at pH <7 by the addition of concentrated hydrochloric acid. The anode solution was treated with sodium sulphite (15 g) and decolourising carbon, allowed to stir for 30 minutes, filtered, and evaporated to low volume. The aqueous residue was extracted with ethyl acetate (2 x 125 ml), the organic extracts combined and washed with saturated sodium chloride solution (2 by 40 ml), dried over magnesium sulphate, and evaporated to dryness. The solid residue was recrystallised from a mixture of ethyl acetate and petroleum ether (bp 60 – 80°) to yield (3S,4S,1′R) – 4 – benzoyl – 3 – (1′ – hydroxyethyl)azetidin – 2 – one as a fawn solid (3.7 g, 39%), m.pt. 130 – 132°.

**Example 18**

(3S,4S,1′R) – 4 – Benzoyl – 3 – (1′ – (t – butyldimethylsilyloxy) – ethyl) – 1 – (p – methoxyphenyl)azetidin – 2 – one (1 g), dissolved in acetonitrile (24 ml) was mixed with a 5% aqueous solution of sodium

tetrafluoroborate (6ml) and placed in the anode compartment of an H-cell. The cathode compartment contained a 5% aqueous solution of sodium tetrafluoroborate (30ml), adjusted to pH1 by the addition of concentrated hydrochloric acid. The mixture was electrolysed for 3 hours at constant current (80mA), at ambient temperature, with the pH maintained at 7 by adding solid sodium bicarbonate. Additional acid was added to the catholyte as necessary to maintain the pH at 1. The organics were extracted with ethyl acetate (30 ml), the organic layer washed with saturated aqueous sodium bicarbonate, dried, and evaporated. After chromatography (mplc) using a gradient from dichloromethane to 6:4 dichloromethane:ether, there was obtained from the appropriate fractions (3S,4S,1'R)-4-benzyl-3-(1'-(t-butyldimethylsilyloxy)ethyl)-azetidin-2-one (0.5 g, 66%).

Nmr (d6-DMSO, 200 MHz): 0.14 (6H, s); 0.94 (9H, s); 1.24 (3H, d, J = 6.3 Hz); 3.25 (1H, m); 4.35 (1H, dq, J = 4.3 and 6.3 Hz); 5.06 (1H, d, J = 2.7 Hz); 6.17 (1H, br); 7.51 (2H, m); 7.65 (1H, m); 8.06 (2H, m).

**Examples 19-22**

Using the same general techniques and substrate as in Example 18 above, with a total volume of 30 ml solution in the anode compartment, the following variants on the electrolysis conditions were carried out. The yields are by hplc relative to a standard solution; TBABS is tetrabutylammonium bisulphate.

| Ex | Electrolyte | Solvent $CH_3CN:H_2O$ Ratio | Current (mA) | Voltage (V) | Time (hr) | Product (% by hplc) | Notes |
|---|---|---|---|---|---|---|---|
| 19 | 5% Na-pTSA | 4:1 | 80* | 25-30 | 4.5 | 66 | 1 |
| 20 | 5% TBABS | 4:1 | 80* | 12-25 | 6.0 | 65 | 2 |
| 21 | 10% $KNO_3$ | 4:1 | 80* | ~10 | 2.5 | 71 | 3 |
| 22 | 10% $NaBF_4$ | 4:1 | <80 | 5-10 | 4.0 | 72 | 4 |

Notes

* Current constant.

1. Higher voltage led to some heating; temperature 31°C at 1hr; cooled to 24°C. 63% of (3S,4S,1'R)-4-benzoyl-3-(1'-(t-butyldimethylsilyloxy)ethyl)azetidin-2-one and 9% of (3S,4S,1'R)-4-benzoyl-3-(1'-hydroxy)ethyl)-azetidin-2-one after chromatography. Electrolyte was sodium p-toluenesulphonate.

2. Further solid electrolyte added at 0.7 hr to improve conductivity. 10% Desilylation by hplc.

3. 4% Desilylation by hplc.

4. Run with reducing current; 1 hr 60 mA, 2.5 hr 40 mA.

**Example 23**

(3S,4S,1'R)-4-Benzoyl-3-(1'-(t-butyldimethylsilyloxy)ethyl)-1-(p-methoxyphenyl)-azetidin-2-one (1 g), dissolved in acetonitrile (24 ml) was mixed in a beaker with a 5% aqueous solution of sodium

tetrafluoroborate (6 ml) and the pH adjusted to 5 with solid bicarbonate. The mixture was electrolysed using platinum electrodes (4 $cm^2$) at a constant current of 80 mA for 3 hours. By hplc a yield of 30% (3S,4S,1′R)-4-benzoyl-3-(1′-(t-butylidimethylsilyloxy)ethyl)-azetidin-2-one was obtained.

**Example 24**

(3S,4S,1′R)-4-Benzoyl-3-(1′-(t-butyldimethylsilyoxy)ethyl)-1-(p-methoxyphenyl)azetidin-2-one (3 g), dissolved in acetonitrile (24 ml) was mixed with a 5% aqueous solution of sodium tetrafluoroborate (6 ml) and placed in the anode compartment of an H-cell. Sodium bicarbonate (3 g) was added to adjust the pH. The cathode compartment contained a 5% aqueous solution of sodium chloride (30 ml), acidified to pH 1 by the addition of concentrated hydrochloric acid. The mixture was electrolysed for 7 hours at ambient temperature, with the current gradually being reduced from an initial value of 100 mA to 20 mA at the end. Extra sodium bicarbonate (1 g) was added at 4 hours. The anolyte was washed out and extracted with ethyl acetate (300 ml), the extract washed with 10% aqueous sodium sulphite (50 ml), 0.5N sodium hydroxide (100 ml), brine (50 ml) and evaporated. After chromatography on silica using a gradient from dichloromethane to 4:1 dichloromethane:ether, approprate fractions were combined and evaporated to give (3S,4S,1′R)-4-benzoyl-3-(1′-(t-butylidimethylsilyloxy)ethyl)azetidin-2-one (1.9 g, 83%).

**Example 25**

(3S,4S,1′R)-4-Benzoyl-3-(1′-(t-butyldimethylsilyloxy)ethyl)-1-(p-methoxyphenyl)azetidin-2-one (1.66 g), dissolved in a mixture of acetone (25 ml) and 25% aqueous sodium tetrafluoroborate (5 ml) containing sodium bicarbonate (1.5 g) was placed in the anode compartment of an H-cell, with 5% aqueous sodium tetrafluoroborate in the cathode compartment. The solution was electrolysed by passing only sufficient current to keep the anode electrode potential below 1.8V (relative to a calomel electrode). After the completion of the process the anolyte was treated with sodium sulphite (0.5 g) in water (20 ml), stirred briefly and extracted with ethyl acetate (30 ml, then 15 ml). The organic extracts were combined, washed with 0.25N sodium hydroxide (10 ml), saturated sodium chloride (10 ml), dried over magnesium sulphate and evaporated. The crystalline residue was triturated with petroleum ether (bp 60-80°,10 ml) containing ethanol (0.5 ml) to yield (3S,4S,1′R)-4-benzoyl-3-(1′-(t-butylidimethylsilyloxy)ethyl)-azetidin-2-one as a fawn solid (0.68 g).

The strength estimated by hplc was 92%, to give a yield of 63%.

**Example 26**

(3S,4S,1′R)-4-Benzoyl-3-(1′-(t-butyldimethylsilyloxy)ethyl)-1-(p-methoxyphenyl)azetidin-2-one (20 g), was treated with a mixture of acetonitrile (240 ml) and 5% aqueous sodium tetrafluoroborate (48 ml), and placed in the anode reservoir of a flow-cell, as in Example 17 above. The cathode reservoir contained 5% aqueous sodium chloride (170 ml), acidified with concentrated hydrochloric acid (30 ml). The anolyte was pumped through the cell at 1000 ml/min, while the catholyte was pumped at 500 ml/min. Solid sodium bicarbonate (5 g) was added to the anode reservoir, and the mixture electrolysed at aninitial current of 840 mA, dropping to 280 mA over 5 hours. Three extra portions of sodium bicarbonate (5 g) were added at 30 minute intervals, and a final portion after completion of the electrolysis at 7 hours. The anode solution was diluted with ethyl acetate (1000 ml) and 10% aqueous sodium sulphite solution (400 ml), and the organic layer separated. After a further extraction with ethyl acetate (500 ml), the organic layers were combined and washed with 0.5N sodium hydroxide (300 ml), saturated sodium chloride solution, dried over magnesium sulphate, and evaporated. The solid residue (16.3 g) was recrystallised from Essochem solvent 30 (a high boiling point petrol) to yield (3S,4S,1′R)-4-benzoyl-3-(1′-(t-butyldimethylsilyloxy)-ethyl)-azetidin-2-one (7 g, 46%).

**Example 27**

(3S,4S,1′R)-4-Benzoyl-3-(1′-hydroxyethyl)-1-(4-methoxy-2-nitrophenyl)azetidin-2-one (300mg) was dissolved in a mixture of acetonitrile (20 ml) and aqeuous sodium tetrafluoroborate (25% w/v, 4 ml) and placed in the anode compartment of an H-cell, together with solid sodium bicarbonate to maintain a constant pH. The cathode compartment contained aqueous sodium tetrafluoroborate (5% w/v, 25 ml), acidified with 2N sulphuric acid. The mixture was electrolysed at a constant voltage of 2V relative to a calomel electrode for 2 hours. Hplc indicated a product yield of 43%. The dark solution was evaporated to

dryness, partitioned between ethyl acetate (30 ml) and brine (20 ml), and the pH adjusted to 4 with hydrochloric acid. The organic layer was separated, and further extractions made (3 x 20 ml ethyl acetate). The combined organic layers were washed with brine, dried (sodium sulphate) and evaporated. Crude material was further purified by chromatography on silica, eluting with ethyl acetate:dichloromethane 1:1, to give (3S,4S,1′R)-4-benzoyl-3-(1′-hydroxyethyl)azetidin-2-one (60 mg, 34%), identified by comparison with the sample of Example 1 above.

**Example 28**

Using the same conditions as in Example 27, (3S,4S,1′R)-1-(3-acetamido-4-methoxyphenyl)-4-benzoyl-3-(1′-hydroxyethyl)azetidin-2-one (250 mg) was electrolysed at reducing current (60 mA to 20 mA) over 2.5 hours, giving an hplc yield of 36%. Work-up and chromatography as in Example 27 gave (3S,4S,1′R)-4-benzoyl-3-(1′-hydroxyethyl)azetidin-2-one (33 mg, 23%), identified by comparison with a standard sample.

**Example 29**

Using the procedure of Example 27 (3S,4S,1′R)-4-benzoyl-3-(1′-hydroxyethyl)-1-(4-hydroxyphenyl)azetidin-2-one (200 mg) was electrolysed at a constant current of 100 mA for 1 hour. Hplc showed a yield of 65%. Work-up by extraction as in Example 27 gave (3S,4S,1′R)-4-benzoyl-3-(1′-hydroxyethyl)azetidin-2-one (105 mg, 75%), identified by comparison with a reference sample.

**Example 30**

Using the conditions of Example 27 (3S,4S,1′R)-4-benzoyl-3-(1′-(t-butyldimethylsilyloxy)ethyl)-1-(3-chloro-4-methoxyphenyl)azetidin-2-one (300 mg) was electrolysed at a constant voltage of 2.1V relative to a calomel electrode for 2 hours, to give an hplc yield of 80%. Work-up as in Example 27 and chromatography gave (3S,4S,1′R)-4-benzoyl-3-(1′-hydroxyethyl)azetidin-2-one (58 mg, 42%), identified by comparison with a reference sample.

**Example 31**

Using the conditions of Example 27, (3S,4S,1′R)-4-benzoyl-1-(3-bromo-4-methoxyphenyl)-3-(1′-hydroxyethyl)azetidin-2-one (300 mg) was electrolysed in the anode compartment at constant current of 60 mA for 1.5 hours, to give an hplc yield of 82%. Work-up as in Example 27 gave (3S,4S,1′R)-4-benzoyl-3-(1′-hydroxyethyl)azetidin-2-one (121 mg, 74%), identified by comparison with a reference sample.

**Example 32**

A solution of (3S,4S,1′R)-3-(1′-(allyloxycarbonyloxy)ethyl)-4-benzoyl-1-(p-methoxyphenyl)-azetidin-2-one (1 g) in acetonitrile (25 ml) was placed in the anode compartment of an H-cell, and mixed with an aqueous solution of sodium tetrafluoroborate (25% w/v, 5 ml) and sodium bicarbonate (3 g). The cathode compartment was filled with aqueous sodium tetrafluoroborate (5% w/v, 30 ml), acidified to pH < 1 with sulphuric acid. The system was electrolysed at a constant potential of 1.35V relative to a standard calomel electrode for 2 hours. The anode contents were diluted with ethyl acetate (200 ml) and water (50 ml). The organic phase was separated, dried (magnesium sulphate) and evaporated. After chromatography on silica, eluting with a gradient from dichloromethane to dichloromethane:ether 70:30, there was obtained (3S,4S,1′R)-3-(1′-(allyloxycarbonyloxy)ethyl)-4-benzoylazetidin-2-one (535 mg, 72%). A sample recrystallised from dichloromethane/hexane had mp 90-91°. Microanalysis: Found: C, 63.2; H, 5.7; N, 4.4%. Calc. for $C_{16}H_{17}NO_5$: C, 63.4; H, 55.6; N, 4.6%
Nmr (CDCl3, 200MHz): 1.48 (3H, d, J = 6.0 Hz); 3.41 (1H, dt, J = 2.8, 7.7 Hz); 4.66 (1H, dt, J = 1.3, 6.0 Hz); 5.0 (1H, d, J = 2.48 Hz); 5.2-5.45 (3H, m); 5.96 (1H, m); 6.29 (1H, brs); 7.47 (2H, m); 7.64 (1H, m); 8.00 (2H, m).

## Example 33

Using the standard conditions of Example 33 above, (3R,4R,1′R)-4-benzoyloxy-3-(1′-(t-butyldimethylsilyloxy)ethyl)-1-(4-methoxyphenyl)azetidin-2-one (1 g) was electrolysed at a constant potential of 1.45 V relative to a standard calomel electrode for 3.5 hours. Aqueous work-up as Example 32 above, followed by chromatography (elution with a gradient from dichloromethane too dichloromethane:ether 90:10) gave (3R,4R,1′R)-4-benzoyloxy-3-(1′-(t-butyldimethylsilyloxy)ethyl)azetidin-2-one (600 mg, 78%). A sample recrystallised from hexane gave mp 123-124°. Microanalysis: Found: C, 62.0; H, 7.8; N, 3.9%. Calc. for $C_{18}H_{27}NO_4Si$: C, 61.9; H, 7.8; N, 4.0%

## Example 34

Using the standard conditions of Example 32 above, (3R,4R,1′R)-4-benzoyloxy-3-(1′-hydroxyethyl)-1-(4-methoxyphenyl)azetidin-2-one (1 g) was electrolysed at a constant potential of 1.38 V relative to a standard calomel electrode for 3 hours. The anode solution was diluted with ethyl acetate (200 ml) and excess of magnesium sulphate (50 g) added, the mixture filtered and evaporated. Purification by chromatography on silica (eluting with a gradient from dichloromethane) to ethyl acetate) gave (3R,4R,1′R)-4-benzoyloxy-3-(1′-hydroxyethyl)azetidin-2-one (474 mg, 69%). A sample recrystallised from isopropanol gave mp 151-153°. Microanalysis: Found: C, 61.2; H, 5.5; N, 5.7%. Calc. for $C_{12}H_{13}NO_4$: C, 61.3; H, 5.6; N, 6.0%

## Example 35

Using the standard conditions of Example 32 above, (3S,4R,1′R)-4-benzoyl-3-(1′-(t-butyldimethylsilyloxy)ethyl)-1-((4-methoxyphenyl)azetidin-2-one (1 g) was electrolysed at a constant electrode potential of 1.4 V relative to a standard calomel electrode for 3 hours. Aqueous work-up as Example 32 above, followed by chromatography (elution with a gradient from dichloromethane to dichloromethane:ether 75:25) gave (3S,4R,1′R)-4-benzoyl-3-(1′-(t-butyldimethylsilyloxy)ethyl)azetidin-2-one (487 mg, 64%). A sample recrystallised from hexane gave mp 102-104°. Microanalysis: Found: C, 64.6; H, 8.2; N, 3.9%. Calc. for $C_{18}H_{27}NO_3Si$: C, 64.8; H, 8.2; N, 4.2%.
Nmr ($CDCl_3$, 200MHz): -0.24 (3H, s); -0.18 (3H, s); 0.71 (9H, s); 1.05 (3H, d, J = 6.7 Hz); 3.70 (1H, t, J = 5.3 Hz); 4.22 (1H, dq, J = 5.3, 6.6 Hz); 5.16 (1H, d, J = 5.3 Hz); 6.19 (1H, brs)); 7.47 (2H, m); 7.60 (1H, m); 7.94 (2H, m).

## Example 36

Using the standard conditions of Example 32 above, (3S,4S,1′R)-4-benzoyl-3-(1′-(benzyloxycarbonyloxy)ethyl)-1-(p-methoxyphenyl)azetidin-2-one (100 mg) was electrolysed at a constant electrode potential of 1.6 V relative to a standard calomel electrode for 3 hours. Aqueous work-up as Example 32 above, followed by chromatography (elution with a gradient from dichloromethane to dichloromethane:ether 75:25) gave (3S,4S,1′R)-4-benzoyl-3-(1′-(benzyloxycarbonyloxy)ethyl)-azetidin-2-one 46 mg, 60%). A sample recrystallised from ethyl acetate/hexane gave mp 88-90°.
Nmr ($CDCl_3$, 200MHz): 1.49 (3H, d, J = 6.3 Hz); 3.40 (1H, dt, J = 2.7, 7.7 Hz); 4.98 (1H, d, J = 2.7 Hz); 5.21 (2H, d, J = 2.0 Hz); 5.28 (1H, dq, J = 6.3, 7.7 Hz); 6.28 (1H, brs); 7.39 (7H, s + m); 7.58 (1H, m); 7.96 (2H, m).

## Example 37

Using the standard conditions of Example 32 above, (3S,4S,1′R)-3-(1′-acetoxyethyl)-4-benzoyl-1-(p-methoxyphenyl)-azetidin-2-one (1.0 g) was electrolysed at a constant electrode potential of 1.25V relative to a standard calomel electrode for 3 hours. Aqueous work-up as Example 32 above, followed by chromatography (elution with a gradient from dichloromethane to dichloromethane:ether 60:40) gave (3S,4S,1′R)-3-(1′-acetoxyethyl)-4-benzoylazetidin-2-one 531 mg, 70%). A sample recrystallised from t-butyl methyl ether:ethyl acetate 10:1 gave mp 135-136°. Microanalysis: Found: C, 64.3; H, 5.8; N, 5.3%. Calc. for $C_{14}H_{15}NO_4$: C, 64.4; H, 5.8; N, 5.4%.
Nmr ($CDCl_3$, 200MHz): 1.42 (3H, d, J = 6.3 Hz); 2.04 (3H, s); 3.36 (1H, dt, J = 2.3, 8.0 Hz); 4.94 (1H, d, J = 2.3 Hz); 5.41 (1H, dq, J = 6.3, 8.0 Hz); 6.33 (1H, brs); 7.51 (2H, m); 7.65 (1H, m); 7.95 (2H, m).

**Example 38**

Using the standard conditions of Example 32 above, (3S,4S,1'R)-4-benzoyl-3-(1'-benzoyloxyethyl)-1-(p-methoxyphenyl)azetidin-2-one (0.5 g) was electrolysed at a constant electrode potential of 1.55V relative to a standard calomel electrode for 2.5 hours. Aqueous work-up as in Example 32 above, followed by chromatography (elution with a gradient from dichloromethane to dichloromethane:ether 75:25) gave (3S,4S,1'R)-4-benzoyl-3-(1'-benzoyloxyethyl)azetidin-2-one 265 mg, 70%). A sample recrystallised from t-butyl methyl ether:hexane 1:2 gave mp 134-135°. Microanalysis: Found: C, 70.2; H, 5.3; N, 4.2%. Calc. for $C_{19}H_{17}NO_4$: C, 70.6; H, 5.3; N, 4.3%.
Nmr ($CDCl_3$, 200MHz): 1.57 (3H, d, J = 6.3 Hz); 3.48 (1H, dq, J = 2.5, 9.0 Hz); 5.03 (1H, d, J = 2.5 Hz); 5.73 (1H, dq, J = 6.3, 9.0 Hz); 6.32 (1H, brs); 7.22 (2H, m); 7.45 (3H, m); 7.52 (1H, m); 7.89 (2H, m); 7.97 (2H, m).

**Example 39**

Using the standard conditions of Example 32 above, (3S,4S,1'R)-4-benzoyl-3-(1'-(2''-methoxyethoxymethoxy)ethyl)-1-(p-methoxyphenyl)-azetidin-2-one (0.5 g) was electrolysed at a constant electrode potential of 1.55V relative to a standard calomel electrode for 3 hours. Aqueous work-up as Example 32 above, followed by chromatography (elution with a gradient from dichloromethane to ether gave (3S,4S,1'R)-4-benzoyl-3-(1'-(2''-methoxyethoxymethoxy)ethyl)-azetidin-2-one (224 mg, 60%) as a gum.
Nmr ($CDCl_3$, 200MHz): 1.32 (3H, d, J = 6.3 Hz); 3.33 (1H, dt, J = 2.7, 6.3 Hz); 3.38 (3H, s); 3.57 (2H, t); 3.75 (2H, t); 4.29 (1H, quintet, J = 6.3 Hz); 4.85 (2H, ABq, J = 7.3 Hz); 5.03 (1H, d, J = 2.7 Hz); 6.31 (1H, brs,); 7.40 (2H, m); 7.64 (1H, m); 8.09 (2H, m).

**Example 40**

Using the standard conditions of Example 32 above, (3S,4S,1'R)-3-(1'-hydroxyethyl)-1-(p-methoxyphenyl)-4-pivaloylazetidin-2-one (0.5 g) (EP-A-276993) was electrolysed at a constant electrode potential of 1.7V relative to a standard calomel electrode for 2 hours. Work-up as Example 33 above, followed by chromatography (elution with a gradient from dichloromethane to ethyl acetate gave (3S,4S,1'R)-3-(1'-hydroxyethyl)-4-pivaloylazetidin-2-one 224 mg, 69%) as a gum.
Nmr ($CDCl_3$, 200 MHz): 1.23 (9H, s); 1.36 (3H, d, J = 6.3 Hz); 3.21 (1H, dt, J = 2.7, 6.3 Hz); 4.25 (1H, quintet, J = 6.3 Hz); 4.58 (1H, d, J = 2.7 Hz); 6.41 (1H, brs).

**PREPARATION OF STARTING MATERIALS**

There are many, many methods of preparing the azetidinone ring system and any appropriate method may be selected to prepare a particular N-substituted azetidinone for use as a starting material for the process of the present invention. Many of such N-substituted azetidinones are known and the others can be prepared by the methods known in the art; for example see the methods of EP-A-181831, GB-A-2144419 and JP 56-142259.

The preparation of some particular azetidinone starting materials is given below.

(A) (3S,4S,1'R)-4-Benzoyl-3-(1'-hydroxyethyl)-1-(p-methoxyphenyl)azetidin-2-one (3.25 g) was suspended in glacial acetic acid (9 ml) and nitric acid (11% v/v, 18 ml) added to ambient temperature with stirring. The mixture was heated to 100° for 1 hour, quenched into water (50 ml) and the organics extracted into ethyl acetate (4 x 50 ml). After washing with sodium bicarbonate and brine, the dried extract (MgSO4) was evaporated. The residue was subjected to chromatography on silica, using a gradient from ether:hexane 70:30 to ether:hexane 90:10. First eluted was (3S,4S,1'R)-4-benzoyl-3-(1'-hydroxyethyl)-1-(4-methoxy-2-nitro-phenyl)azetidin-2-one as a non-crystalline foam (1.94 g, 53%). Microanalysis: Found: C, 61.5; H,5.0; N, 7.2%. Calc. for $C_{19}H_{18}N_2O_6$ : C, 61.6; H,4.9; N, 7.6%.
Nmr ($CDCl_3$, 200MHz): 1.42 (3H, d, J = 6.3 Hz); 3.27 (1H, dd, J = 2.7, 7.0 Hz); 3.85 (3H, s); 4.45 (1H, quintet, J = 7.0 Hz); 5.82 (1H, d, J = 2.7 Hz); 7.18 (1H, dd, J = 3.0, 9.3 Hz); 7.41 (1H, d, J = 3.0 Hz); 7.48 (2H, m); 7.63 (1H, m); 7.95 (1H, d, J = 9.3 Hz); 8.23 (2H, m).

(B) A solution of the product of (A) above (515 mg) in a mixture of glacial acetic acid (10 ml) and water (3 ml) was treated with activated iron powder (1.5 g), and stirred at 85° for 20 min. The mixture was quenched with water and isolated by conventional extraction into ethyl acetate as in (a) above. The crude

product was dissolved in ethyl acetate (10 ml), cooled in ice and treated with acetic anhydride (0.3 ml) and pyridine (0.24 ml). After stirring for 1 hour, a work-up as in (a) above gave a crude material further purified by chromatography on silica, eluting with ethyl acetate:dichloromethane 1:1, to give (3S,4S,1'R)-1-(3-acetamido-4-methoxyphenyl)-4-benzoyl-3-(1'-hydroxyethyl)azetidin-2-one as a non-crystalline foam (351 mg, 66%). Microanalysis: Found: C, 65.1; H,5.9; N, 6.9; $H_2O$, 3.3%. Calc. for $C_{21}H_{22}N_2O_5$. 0.25 $H_2O$ : C, 65.2; H,5.8; N, 7.2; $H_2O$, 1.2%.

Nmr ($CDCl_3$, 200MHz): 1.35 (3H, d J = 6.3 Hz); 2.12 (3H, s); 3.17 (1H, dd, J = 2.7, 6.7 Hz); 3.84 (3H, s); 4.35 (1H, quintet, J = 6.3 Hz); 5.59 (1H, d, J = 2.7 Hz); 6.80 (1H, d, J = 8.7 Hz); 7.37 (1H, dd, J = 2.7, 8.7 Hz); 7.49 (2H, m); 7.62 (1H, m); 7.70 (1H, br s); 8.02 (1H, d J = 8.7 Hz); 8.18 (2H, m).

(C) A solution of (3S,4S,1'R)-4-benzoyl-3-(1'-hydroxyethyl)-1-(4-methoxyphenyl)azetidin-2-one (650 mg) in dichloromethane (10 ml) was cooled to -40° under argon and treated with 1M boron tribromide in dichloromethane (10 ml). After stirring for 20 minutes, the temperature was allowed to come to ambient over 2 hours. The reaction was quenched in ice and ethyl acetate (50 ml) and solid sodium bicarbonate added to adjust the pH to 7. Ethylene glycol (10 ml) was added and the cold mixture stirred 2 hours before filtering through diatomaceous earth. The organic layer was seperated, further extractions made (3 x 50 ml ethyl acetate), and the combined organic layers worked up in standard manner. Purification by chromatography on silica (eluting with ethyl acetate:dichloromethane 3:1) gave (3S,4S,1'R)-4-benzoyl-3-(1'-hydroxyethyl)-1-(4-hydroxyphenyl)azetidin-2-one as a non-crystalline foam (430 mg, 69%). Microanalysis: Found: C, 68.9; H,5.6; N, 4.1%. Calc. for $C_{18}H_{17}NO_4$: C, 69.4; H,5.5; N, 4.5%.

Nmr (d6-DMSO, 200MHz): 1.16 (3H, d, J = 6.3 Hz); 3.08 (1H, dd, J = 2.7, 6.3 Hz); 4.09 (1H, m); 5.50 (1H, br d, J = 5.0 Hz); 5.75 (1H, d, J = 2.7 Hz); 6.71 (2H, d, J = 9.0 Hz); 7.07 (2H, d, J = 9.0 Hz); 7.61 (2H, m); 7.74 (1H, m); 8.22 (2H, m); 9.27 (1H, s).

(D) A solution of (3S,4S,1'R)-4-benzoyl-3-(1'-(t-butyldimethylsilyloxy)- ethyl)-1-(p-methoxyphenyl)azetidin-2-one (2 g) in acetonitrile (25 ml) was placed in the anode compartment of an H-cell and treated with a solution of tetraethylammonium chloride (1.5 g) in water (5 ml), followed by solid sodium bicarbonate (2 g). The cathode compartment was filled with a solution of tetraethylammonium chloride (3 g) in water (30 ml), acidified with hydrochloric acid. The mixture was electrolysed for 5.5 hours at a constant voltage of 1.1V relative to a calomel electrode. The anode mixture was evaporated, partitioned between ethyl acetate (50 ml) and water (30 ml) and the organic layer separated. After two further extractions (20 ml ethyl acetate), the combined organic layers were dried and evaporated. After chromatography on silica, eluting with dichloromethane, (3S,4S,1'R)-4-benzoyl-3-(1'-(t-butyldimethylsilyloxy)ethyl)-1-(3-chloro-4-methoxyphenyl)azetidin-2-one was obtained (1.32 g, 61%). Recrystallisation from hexane gave mp 115°. Microanalysis: Found: C, 63.6; H, 6.9; N, 2.9%. Calc. for $C_{25}H_{32}ClNO_4Si$: C, 63.4; H, 6.8; N, 3/0%.

Nmr ($CDCl_3$, 200MHz): 0.06 (3H, s); 0.11 (3H, s); 0.83 (9H, s); 1.20 (3H, d, J = 6.3 Hz); 3.20 (1H, t, J = 2.8 Hz); 3.84 (3H, s); 4.41 (1H, dq, J = 3.0, 6.3 Hz); 5.56 (1H, d, J = 3.0 Hz); 6.82 (1H, d, J = 9.3 Hz); 7.13 (1H, dd, J = 2.7, 9.3 Hz); 7.32 (1H, d, J = 2.7 Hz); 7.54 (2H, m); 7.67 (1H, m); 8.08 (2H, m).

(E) (3S,4S,1'R)-4-Benzoyl-3-(1'-hydroxyethyl)-1-(4-methoxyphenyl)-azetidin-2-one (2 g) was suspended in acetonitrile (25 ml) in the anode compartment of an H-cell, treated with tetraethylammonium bromide (2.6 g) in water (5 ml) and solid sodium bicarbonate (2 g). The cathode compartment was filled with a solution of tetraethylammonium bromide (3 g) in water (30 ml), acidified with hydrobromic acid. The system was electrolysed at a constant voltage of 1.2V relative to a calomel electrode for 6.5 hours. Work-up as in (D) above, followed by chromatography on silica, eluting with dichloromethane: ethyl acetate 9:1, gave (3S,4S,1'R)-4-benzoyl-1-(3-bromo-4-methoxyphenyl)-3-(1'-hydroxyethyl)-azetidin-2-one (1.67 g, 67%), as a foam. Microanalysis: Found: C, 55.9; H, 4.6; N, 3.2%. Calc. for $C_{19}H_{18}BrNO_4$: C, 56.4; H, 4.5; H, 3.5%.

Nmr ($CDCl_3$, 200MHz): 1.37 (3H, d, J = 6.3 Hz); 3.22 (1H, dd, J = 2.3, 6.3 Hz); 3.85 (3H, s); 4.38 (1H, quintet, J = 6.3 Hz); 5.53 (1H, d, J = 2.3 Hz); 6.80 (1H, d, J = 8.8 Hz); 7.16 (1H, dd, J = 2.5, 8.8 Hz); 7.55 (3H, m); 7.68 (1H, m); 8.20 (2H, m).

(F) A solution of (3S,4S,1'R)-4-benzoyl-3-(1'-(t-butyldimethylsilyloxy)ethyl)-1-(4-methoxyphenyl)azetidin-2-one (2.92 g) in ethyl acetate (20 ml) was treated with anhydrous sodium acetate (100 mg) and 40% peracetic acid (3.14 ml). The mixture was stirred at ambient temperature for 25 hours. The mixture was diluted with ethyl acetate (50 ml), washed with 10% aqueous sodium sulphite, 5% aqueous sodium bicarbonate, dried (magnesium sulphate) and evaporated. The product was purified by chromatography on silica, eluting with a gradient from hexane to hexane:ethyl acetate 70:30, to give (3R,4R,1'R)-4-benzoyloxy-3-(1'-(t-butyldimethylsilyloxy)ethyl)-1-(4-methoxyphenyl)azetidin-2-one as an oil (2.7 g, 89%).

Nmr ($CDCl_3$, 200MHz): 0.03 (3H, s); 0.10 (3H, s); 0.78 (9H, s); 1.40 (3H, d, J = 6.3 Hz); 3.35 (1H, dd, J = 1.0, 2.7 Hz); 3.79 (3H, s); 4.39 (1H, dq, J = 2.7, 6.3 Hz); 6.35 (3H, m); 7.42 (2H, d, J = 9.3 Hz); 7.47 (2H, m); 7.63 (1H, m); 8.07 (2H, m).

(G) (3S,4S,1′R)-4-Benzoyl-3-(1′-hydroxyethyl)-1-(4-methoxyphenyl)-azetidin-2-one (present largely as the lactol form in the solid state, 4.05 g) was treated under argon with a mixed solution in dimethylformamide (35 ml) of t-butyldimethylsilylchloride (0.5 M) and imidazole (1.0 M) and stirred at room temperature for 16 hours. After evaporation to dryness, the residue was diluted with water (100 ml) and extracted into ether (150 ml). After washing with aqueous sodium dihydrogen phosphate (10%, 50 ml), and brine, the dried (magnesium sulphate) residue was evaporated. Purification by chromatography on silica, eluting with dichloromethane, gave (3S,4R,1′R)-4-benzoyl-3-(1′-(t-butyldimethylsilyloxy)-ethyl)-1-(4-methoxyphenyl)azetidin-2-one (3.61 g, 66%). A sample recrystallised from cyclohexane gave mp 82-83°. Microanalysis: Found: C, 68.3; H, 7.6; N, 3.2%. Calc. for $C_{25}H_{33}NO_4Si$: C, 68.3; H, 7.6: N, 3.1%.

Nmr ($CDCl_3$, 200MHz): -0.40 (3H, s); -0.19 (3H, s); 0.66 (9H, s); 1.30 (3H, d, J = 6.3 Hz); 3.71 (1H, dd, J = 5.7, 7.7 Hz); 3.74 (3H, s); 4.29 (1H, dq, J =6.3, 7.7 Hz); 5.56 (1H, d, J = 5.7 Hz); 6.78 (2H, d, J = 9 Hz); 7.14 (2H, d J = 9 Hz); 7.50 (2H, m); 7.64 (1H, m); 8.04 (2H, m).

(H) A solution of (3S,4S,1′R)-4-benzoyl-3-(1′-hydroxyethyl)-1-(p-methoxyphenyl)azetidin-2-one (4.88 g) in dichloromethane (50 ml) under argon was treated with 4-dimethylaminopyridine (7.32 g) and triethylamine (8.4 ml) and cooled in ice. To this was added benzyloxycarbonyl chloride (12,8 ml), and the mixture allowed to come to ambient temperature. After stirring for 60 hours, the mixture was diluted with water (50 ml), the organic layer separated, a further extraction made with dichloromethane (50 ml), and the combined extracts dried over magnesium sulphate. After purification by chromatography, using a gradient from dichloromethane to dichloromethane:ethyl acetate 1:1, there was obtained (3S,4S,1′R)-4-benzoyl-3-(1′-(benzyloxycarbonyloxy)ethyl)-1-(p-methoxyphenyl)azetidin-2-one (5.13 g, 74%). Recrystallisation from hexane/ethyl acetate gave a mp of 139-141°. Microanalysis: Found: C, 70.8; H, 5.4; N, 2.9%. Calc. for $C_{27}H_{25}NO_6$: C, 70.6; H, 5.5; H, 3.0%.

Nmr ($CDCl_3$, 200MHz): 1.52 (3H, d, J = 6.3 Hz); 3.46 (1H, dd, J = 2.6, 8.7 Hz); 3.76 (3H, s); 5.19 (2H, ABq, J = 12.0 Hz); 5.30 (1H, dq, J = 6.3, 8.3 Hz); 5.47 (1H, d, J = 2.6 Hz); 6.82 (2H, d, J = 9.3 Hz); 7.19 (2H, d, J = 9.3 Hz); 7.40 (7H, m); 7.60 (1H, m); 8.07 (2H, m).

(I) A solution of (3S,4S,1′R)-4-benzoyl-3-(1′-hydroxyethyl)-1-(p-methoxyphenyl)azetidin-2-one (7 g) in dichloromethane (30 ml) was treated successively with pyridine (5.2 ml) and then, dropwise, with acetyl chloride (2.3 ml). The mixture was stirred at room temperature for 1 hour, then quenched with water, and the organics layers separated. After a further extraction with dichloromethane (50 ml), the combined organic extracts were washed with 2M hydrochloric acid, water, then brine, and dried (sodium sulphate). After evaporation of the solvent, the material was purified by chromatography on silica, eluting with dichloromethane:ethyl acetate 85:15, to give (3S,4S,1′R)-3-(1′-acetoxyethyl)-4-benzoyl-1-(p-methoxyphenyl)azetidin-2-one (7.24 g, 91%), as a solid, mp 91-93°.

Nmr ($CDCl_3$, 200MHz): 1.46 (3H, d, J = 6.3 Hz); 1.97 (3H, s); 3.34 (1H, dd, J = 2.7, 9.0 Hz); 3.78 (3H, s); 5.31 (1H, d, J = 2.7 Hz); 5.45 (1H, dq, J = 6.3, 9.0 Hz); 6.85 (2H, d, J = 9.3 Hz); 7.21 (2H, d, J = 9.3 Hz); 7.56 (2H, m); 7.71 (1H, m); 8.07 (2H, m).

(J) A solution of (3S,4S,1′R)-4-benzoyl-3-(1′-hydroxyethyl)-1-(p-methoxyphenyl)azetidin-2-one (5 g) in dichloromethane (40 ml), was treated with pyridine (2.6 ml), followed by the dropwise addition of benzoyl chloride (3.6 ml). After stirring for 16 hours at ambient temperature, the mixture was quenched into water, and product isolated as in (I) above. After chromatography using dichloromethane:ethyl acetate 95:5 as eluant, there was obtained (3S,4S,1′R)-4-benzoyl-3-(1′-benzoyloxyethyl)-1-(p-methoxyphenyl)azetidin-2-one as a non-crystalline foam (6.3 g, 95%).

Nmr ($CDCl_3$, 200MHz): 1.51 (3H, d, J = 6.3 Hz); 3.47 (1H, dd, J = 2.3, 9.3 Hz); 3.77 (3H, s); 5.49 (1H, d, J = 2.3 Hz); 5.73 (1H, dq, J = 6.3, 9.3 Hz); 6.84 (2H, d, J = 9.7 Hz); 7.22 (4H, m); 7.3-7.6 (4H, m); 7.90 (4H, m).

(K) A solution of (3S,4S,1′R)-4-benzoyl-3-(1′-hydroxyethyl)-1-(p-methoxyphenyl)azetidin-2-one (1 g) in dichloromethane (15 ml) was treated successively with ethyldiisopropylamine (1.33 ml) and 2-methoxyethoxymethyl chloride (0.92 ml). The mixture was stirred at ambient temperature for 16 hours, then quenched into water, and worked up as in (I) above. The crude product was purified by chromatography on silica to give (3S,4S,1′R)-4-benzoyl-3-(1′-(2″-methoxyethoxymethoxy)ethyl)-1-(p-methoxyphenyl)azetidin-2-one as a foam (1.2 g, 94%).

Nmr ($CDCl_3$, 200MHz): 1.32 (3H, d, J = 6.3 Hz); 3.27 (1H, dd, J = 2.3, 6.3 Hz); 3.36 (3H, s); 3.56 (2H, m); 3.72 (2H, m); 3.77 (3H, s); 4.33 (1H, quintet, J = 6.3 Hz); 4.83 (2H, ABq, J = 7.0 Hz); 5.52 (1H, d, J = 2.3 Hz); 6.82 (2H, d, J = 9.0 Hz); 7.21 (2H, d, J = 9.0 Hz); 7.53 (2H, m); 7.67 (1H, m); 8.16 (2H, m).

## Claims

1. A process for preparing an azetidin-2-one wherein the ring nitrogen atom carries a hydrogen atom which process comprises electrochemically oxidising an azetidin-2-one wherein the ring nitrogen atom is substituted by a phenyl group carrying an ortho- or para-substituent selected from hydroxy, optionally substituted alkoxy, optionally substituted phenoxy, alkenyloxy, protected hydroxy, amino, $C_{1-6}$alkylamino, di-$C_{1-6}$alkylamino, arylamino, di-arylamino, acylamino or di-acylamino, in a liquid medium which comprises water and optionally an organic solvent substantially inert to electrolytic oxidation, in the presence of a working electrode at a potential effective to oxidise and subsequently remove the N-substituent without undesired substantial oxidation of the remainder of the molecule.

2. A process according to claim 1 for preparing a compound of the formula I:

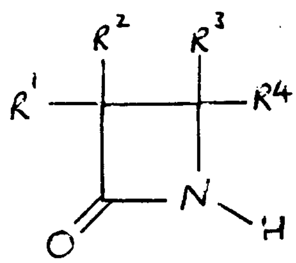

wherein $R^1$ and $R^2$ are independently hydrogen, optionally substituted $C_{1-6}$ alkyl, optionally substituted $C_{2-6}$ alkenyl, optionally substituted $C_{2-6}$alkynyl, $C_{3-8}$cycloalkyl, aryl, optionally substituted $C_{1-6}$alkoxy, halo, optionally substituted $C_{2-6}$alkenyloxy, azido, acylamino, hydroxy, $C_{1-6}$alkanoyloxy, $C_{1-6}$alkanoyl, arylcarbonyl, arylcarbonyloxy, $C_{1-6}$alkylthio, $C_{1-6}$alkyldithio, arylthio, carboxy, $C_{1-6}$alkoxycarbonyl or aryloxycarbonyl, wherein aryl is optionally substituted phenyl; and optional substituents include $C_{1-6}$alkyl, $C_{2-6}$alkenyl, phenyl, heteroaryl or heterocyclyl wherein the heteroatoms are selected from 1-4 oxygen, sulphur or nitrogen atoms, hydroxy, $C_{1-6}$ alkoxy, $C_{1-6}$alkylamino, di-$C_{1-6}$alkylamino, $C_{1-6}$-alkylthio, sulphamoyl, ureido, amidino, guanidino, bromo, chloro, fluoro, cyano, carboxy and nitro;

and $R^3$ and $R^4$ are independently hydrogen, optionally substituted $C_{1-6}$ alkyl, optionally substituted $C_{2-6}$ alkenyl, optionally substituted $C_{2-6}$alkynyl, $C_{3-8}$cycloalkyl, aryl, optionally substituted $C_{1-6}$alkoxy, halo, optionally substituted $C_{2-6}$alkenyloxy, azido, acylamino, di-$C_{1-6}$alkoxyphosphinyl, $C_{1-6}$alkanoyloxy, $C_{1-6}$-alkanoyl, arylcarbonyl, arylcarbonyloxy, $C_{1-6}$alkylthio, $C_{1-6}$-alkyldithio, aryl-thio, carboxy, $C_{1-6}$alkoxycarbonyl, $C_{1-6}$alkanoylthio, arylcarbonylthio and aryloxycarbonyl and the like wherein aryl is optionally substituted phenyl; and optional substituents include $C_{1-6}$-alkyl, $C_{2-6}$alkenyl, phenyl, heteroaryl or heterocyclyl wherein the heteroatoms are selected from 1-4 oxygen, sulphur or nitrogen atoms, hydroxy, $C_{1-6}$alkoxy, $C_{1-6}$alkylamino, di-$C_{1-6}$alkylamino, $C_{1-6}$-alkylthio, sulphamoyl, ureido, amidino, guanidino, bromo, chloro, fluoro, cyano, carboxy, nitro and the like; any functional group being protected by a protecting group if desired.

3. A process according to claim 2 for preparing a compound wherein $R^1$ is 1-hydroxyethyl or 1-protected hydroxyethyl, $R^2$ and $R^3$ are independently hydrogen and $R^4$ is carboxy, $C_{1-6}$alkoxycarbonyl, $C_{1-6}$ alkanoyl, arylcarbonyl, arylcarbonyloxy or $C_{1-6}$alkanoyloxy.

4. A process according to any one of claims 1 to 3 wherein the azetidinone ring nitrogen atom is substituted by a group of the formula II:

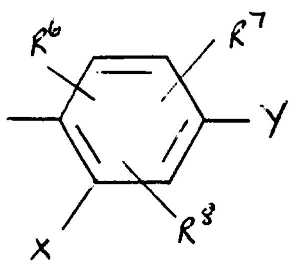

wherein one of X and Y is a group $-OR^5$ wherein $R^5$ is hydrogen, a hydroxy protecting group, optionally substituted $C_{1-6}$alkyl, optionally substituted phenyl or $C_{2-6}$alkenyl, the other of X and Y is a group $R^9$ and $R^6-R^9$ are selected from hydrogen, hydroxy, protected hydroxy, optionally substituted $C_{1-6}$alkyl, optionally substituted $C_{1-6}$alkoxy, optionally substituted phenoxy, $C_{2-6}$alkenyloxy, halo, aryl and heteroaryl.
or is substituted by a group of the formula III:

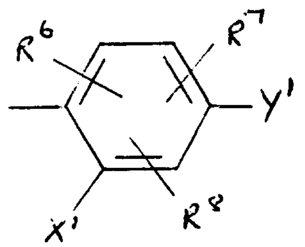

wherein one of X' and Y' is a group $-NR^{10}R^{11}$ wherein $R^{10}$ and $R^{11}$ are independently hydrogen, $C_{1-6}$alkyl for example methyl, aryl for example phenyl or acyl for example $C_{1-6}$alkanoyl such as acetyl, the other of X' and Y' is a group $R^9$ and $R^6-R^9$ are as defined above.

**5.** A process according to claim 1 wherein the azetidinone is 4-benzoyl-3-(1-hydroxyethyl)-1-(p-methoxyphenyl)azetidin-2-one or a protected hydroxy derivative thereof.

**6.** A process according to any one of claims 1 to 5 wherein the liquid medium comprises up to about 50% water (vol/vol) and a substantially inert organic solvent, the working electrode substantially comprises platinum, carbon, silver oxide, manganese oxide, iron oxide, gold, ruthenium, iridium or rhodium and the process is performed at a temperature in the range of about $-5\,^\circ C$ to about $+85\,^\circ C$.

**7.** A process according to any one of claims 1 to 6 wherein the working electrode is platinum or platinised titanium.

**8.** A process according to any one of claims 1 to 7 wherein the potential of the working electrode is in the range 1.2-1.8 Volt relative to a calomel reference electrode.

**9.** A process according to any one of claims 1 to 8 wherein the pH is maintained in the range 4 to 7.

**Patentansprüche**

**1.** Verfahren zur Herstellung eines Azetidin-2-ons, dessen Ringstickstoffatom ein Wasserstoffatom trägt, bei welchem Verfahren elektrochemisch ein Azetidin-2-on, dessen Ringstickstoffatom durch eine Phenylgruppe substituiert ist, die einen ortho- oder para-Substituenten trägt, welcher ausgewählt ist aus Hydroxy, gegebenenfalls substituiertem Alkoxy, gegebenenfalls substituiertem Phenoxy, Alkenyloxy, geschütztem Hydroxy, Amino, $C_{1-6}$Alkylamino, Di-$C_{1-6}$alkylamino, Arylamino, Diarylamino, Acylamino oder Diacylamino, in einem flüssigen Medium, das Wasser und gegebenenfalls ein gegenüber elektrolytischer Oxidation im wesentlichen inertes organisches Lösungsmittel enthält, in Gegenwart einer Arbeitselektrode bei einem Potential oxidiert wird, das eine Oxidation und anschließende Entfernung des N-Substituenten bewirkt, ohne daß eine unerwünschte wesentliche Oxidation des Restes des Moleküls eintritt.

**2.** Verfahren nach Anspruch 1 zur Herstellung einer Verbindung der Formel I,

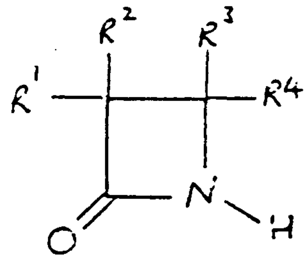

I

bei welcher
$R^1$ und $R^2$ unabhängig für Wasserstoff, gegebenenfalls substituiertes $C_{1-6}$Alkyl, gegebenenfalls substituiertes $C_{2-6}$Alkenyl, gegebenenfalls substituiertes $C_{2-6}$Alkinyl, $C_{3-8}$Cycloalkyl, Aryl, gegebenenfalls substituiertes $C_{1-6}$Alkoxy, Halogeno, gegebenenfalls substituiertes $C_{2-6}$Alkenyloxy, Azido, Acylamino, Hydroxy, $C_{1-6}$Alkanoyloxy, $C_{1-6}$Alkanoyl, Arylcarbonyl, Arylcarbonyloxy, $C_{1-6}$Alkylthio, $C_{1-6}$Alkyldithio, Arylthio, Carboxy, $C_{1-6}$Alkoxycarbonyl oder Aryloxycarbonyl stehen, wobei Aryl gegebenenfalls substituiertes Phenyl bedeutet und die fakultativen Substituenten ausgewählt sind aus $C_{1-6}$Alkyl, $C_{2-6}$Alkenyl, Phenyl, Heteroaryl oder Heteroarylcyclyl mit 1 bis 4 Sauerstoff-, Schwefel- oder Stickstoffatomen als Heteroatome, Hydroxy, $C_{1-6}$Alkoxy, $C_{1-6}$Alkylamino, Di-$C_{1-6}$alkylamino, $C_{1-6}$Alkylthio, Sulfamoyl, Ureido, Amidino, Guanidino, Bromo, Chloro, Fluoro, Cyano, Carboxy und Nitro; und
$R^3$ und $R^4$ unabhängig für Wasserstoff, gegebenenfalls substituiertes $C_{1-6}$Alkyl, gegebenenfalls substituiertes $C_{2-6}$Alkenyl, gegebenenfalls substituiertes $C_{2-6}$-Alkyinyl, $C_{3-8}$-Cycloalkyl Aryl, gegebenenfalls substituiertes $C_{1-6}$Alkoxy, Halogeno, gegebenenfalls substituiertes $C_{2-6}$Alkenyloxy, Azido, Acylamino, Di-$C_{1-6}$alkoxyphosphinyl, $C_{1-6}$Alkanoyloxy, $C_{1-6}$Alkanoyl, Arylcarbonyl, Arylcarbonyloxy, $C_{1-6}$Alkylthio, $C_{1-6}$Alkyldithio, Arylthio, Carboxy, $C_{1-6}$Alkoxycarbonyl, $C_{1-6}$Alkanoylthio, Arylcarbonylthio und Aryloxycarbonyl und dergleichen, wobei Aryl gegebenenfalls substituiertes Phenyl bedeutet und die fakultativen Substituenten ausgewählt sind aus $C_{1-6}$Alkyl, $C_{2-6}$Alkenyl, Phenyl, Heteroaryl oder Heteroarylcyclyl mit 1 bis 4 Sauerstoff-, Schwefel- oder Stickstoffatomen als Heteroatome, Hydroxy, $C_{1-6}$Alkoxy, $C_{1-6}$Alkylamino, Di-$C_{1-6}$alkylamino, $C_{1-6}$Alkylthio, Sulfamoyl, Ureido, Amidino, Guanidino, Bromo, Chloro, Fluoro, Cyano, Carboxy, Nitro und dergleichen ;
wobei jegliche funktionellen Gruppen gegebenenfalls durch eine Schutzgruppe geschützt sind.

**3.** Verfahren nach Anspruch 2 zur Herstellung einer Verbindung, bei welcher $R^1$ für 1-Hydroxyethyl oder geschütztes 1-Hydroxyethyl steht, $R^2$ und $R^3$ jeweils für Wasserstoff stehen und $R^4$ für Carboxy, $C_{1-6}$Alkoxycarbonyl, $C_{1-6}$Alkanoyl, Arylcarbonyl, Arylcarbonyloxy oder $C_{1-6}$Alkanoyloxy steht.

**4.** Verfahren nach einem der Ansprüche 1 bis 3, bei welchem das Azetidinon-Ringstickstoffatom substituiert ist durch eine Gruppe der Formel II,

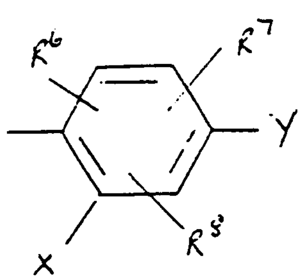

II

worin eines der Symbole X und Y für eine Gruppe $OR^5$ steht, worin $R^5$ Wasserstoff, eine Hydroxyschutzgruppe, gegebenenfalls substituiertes $C_{1-6}$Alkyl, gegebenenfalls substituiertes Phenyl oder $C_{2-6}$Alkenyl bedeutet, und das andere der Symbole X und Y für eine Gruppe $R^9$ steht und $R^6$ bis $R^9$ ausgewählt sind aus Wasserstoff, Hydroxy, geschütztem Hydroxy, gegebenenfalls substituiertem $C_{1-6}$ Alkyl, gegebenenfalls substituiertem $C_{1-6}$Alkoxy, gegebenenfalls substituiertem Phenoxy, $C_{2-6}$Alkenyloxy, Halogeno, Aryl und Heteroaryl,

oder substituiert ist durch eine Gruppe der Formel III,

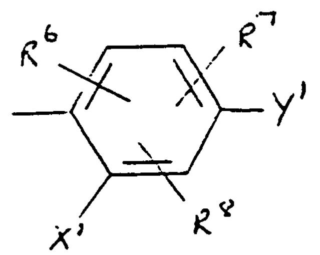

III

worin eines der Symbole X' und Y' für eine Gruppe $-NR^{10}R^{11}$ steht, worin $R^{10}$ und $R^{11}$ unabhängig Wasserstoff, $C_{1-6}$Alkyl, z.B. Methyl, Aryl, z.B. Phenyl, oder Acyl, z.B. $C_{1-6}$Alkanoyl, beispielsweise Acetyl, bedeuten, und das andere der Symbole X' und Y' für eine Gruppe $R^9$ steht und $R^6$ bis $R^9$ die oben angegebenen Bedeutungen besitzen.

**5.** Verfahren nach Anspruch 1, bei welchem das Azetidinon aus 4-Benzoyl-3-(1-hydroxyethyl)-1-(p-methoxyphenyl)azetidin-2-on oder einem geschützten Hydroxyderivat davon besteht.

**6.** Verfahren nach einem der Ansprüche 1 bis 5, bei welchem das flüssige Medium aus bis zu ungefähr 50 Vol.% Wasser und aus einem im wesentlichen innerten organischen Lösungsmittel besteht, die Arbeitselektrode im wesentlichen aus Platin, Kohlenstoff, Silberoxid, Manganoxid, Eisenoxid, Gold, Ruthenium, Iridium oder Rhodium besteht und das Verfahren bei einer Temperatur im Bereich von ungefähr $-5$ °C bis ungefähr $+85$ °C ausgeführt wird.

**7.** Verfahren nach einem der Ansprüche 1 bis 6, bei welchem die Arbeitselektrode aus Platin oder platinisiertem Titan besteht.

**8.** Verfahren nach einem der Ansprüche 1 bis 7, bei welchem das Potential der Arbeitselektrode im Bereich von 1,2 bis 1,8 V, bezogen auf eine Calomel-Bezugselektrode, liegt.

**9.** Verfahren nach einem der Ansprüche 1 bis 8, bei welchem der pH im Bereich von 4 bis 7 gehalten wird.

## Revendications

**1.** Procédé de production d'une azétidine-2-one dont l'atome d'azote du noyau porte un atome d'hydrogène, procédé qui consiste à oxyder par voie électrochimique une azétidine-2-one dont l'atome d'azote du noyau est substitué par un groupe phényle portant un substituant, en position ortho ou para, pouvant être un groupe hydroxy, alkoxy facultativement substitue, phénoxy facultativement substitué, alcényloxy, hydroxy protégé, amino, (alkyle en $C_1$ à $C_6$)amino, di-(alkyle en $C_1$ à $C_6$)amino, arylamino, di-arylamino, acylamino ou di-acylamino, dans un milieu liquide qui comprend de l'eau et, facultativement, un solvant organique principalement inerte vis-à-vis d'une oxydation électrolytique, en présence d'une électrode de travail à un potentiel qui convient pour oxyder puis éliminer le substituant de l'azote sans oxydation notable non désirée du reste de la molécule.

**2.** Procédé suivant la revendication 1, pour la production d'un composé de formule I :

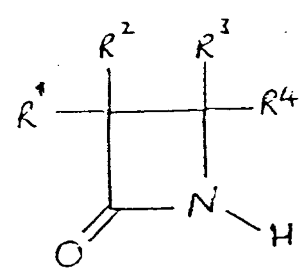

dans laquelle $R^1$ et $R^2$ représentent indépendamment l'hydrogène, un groupe alkyle en $C_1$ à $C_6$ facultativement substitué, alcényle en $C_2$ à $C_6$ facultativement substitué, alcynyle en $C_2$ à $C_6$ facultativement substitué, cycloalkyle en $C_3$ à $C_8$, aryle, alkoxy en $C_1$ à $C_6$ facultativement substitué, halogéno, alcényloxy en $C_2$ à $C_6$ facultativement substitué, azido, acylamino, hydroxy, alcanoyloxy en $C_1$ à $C_6$, alcanoyle en $C_1$ à $C_6$, arylcarbonyle, arylcarbonyloxy, alkylthio en $C_1$ à $C_6$, alkyldithio en $C_1$ à $C_6$, arylthio, carboxy, (alkoxy en $C_1$ à $C_6$)carbonyle ou aryloxycarbonyle, dont le groupe aryle est un groupe phényle facultativement substitué ; et les substituants facultatifs comprennent des substituants alkyle en $C_1$ à $C_6$, alcényle en $C_2$ à $C_6$, phényle, hétéroaryle ou hétérocyclyle dont les hétéroatomes sont choisis parmi 1 à 4 atomes d'oxygène, de soufre ou d'azote, hydroxy, alkoxy en $C_1$ à $C_6$, alkylamino en $C_1$ à $C_6$, di(alkyle en $C_1$ à $C_6$)amino, alkylthio en $C_1$ à $C_6$, sulfamoyle, uréido, amidino, guanidino, bromo, chloro, fluoro, cyano, carboxy et nitro ;

et $R^3$ et $R^4$ représentent indépendamment l'hydrogène, un groupe alkyle en $C_1$ à $C_6$ facultativement substitué, alcényle en $C_2$ à $C_6$ facultativement substitué, alcynyle en $C_2$ à $C_6$ facultativement substitué, cycloalkyle en $C_3$ à $C_8$, aryle, alkoxy en $C_1$ à $C_6$ facultativement substitué, halogéno, alcényloxy en $C_2$ à $C_6$ facultativement substitué, azido, acylamino, di(alkoxy en $C_1$ à $C_6$)phosphinyle, alcanoyloxy en $C_1$ à $C_6$, alcanoyle en $C_1$ à $C_6$, arylcarbonyle, arylcarbonyloxy, alkylthio en $C_1$ à $C_6$, alkyldithio en $C_1$ à $C_6$, arylthio, carboxy, (alkoxy en $C_1$ à $C_6$)carbonyle, alcanoylthio en $C_1$ à $C_6$, arylcarbonylthio et aryloxycarbonyle, etc, le groupe aryle étant un groupe phényle facultativement substitué ; et des substituants facultatifs comprennent des groupes alkyle en $C_1$ à $C_6$, alcényle en $C_2$ à $C_6$, phényle, hétéroaryle ou hétérocyclyle dont les hétéroatomes sont choisis parmi 1 à 4 atomes d'oxygène, de soufre ou d'azote, hydroxy, alkoxy en $C_1$ à $C_6$, (alkyle en $C_1$ à $C_6$)amino, di(alkyle en $C_1$ à $C_6$)amino, alkylthio en $C_1$ à $C_6$, sulfamoyle, uréido, amidino, guanidino, bromo, chloro, fluoro, cyano, carboxy, nitro, etc ; tout groupe fonctionnel étant protégé éventuellement par un groupe protecteur.

**3.** Procédé suivant la revendication 2 pour la préparation d'un composé dans lequel $R^1$ est un groupe 1-hydroxyéthyle ou 1-hydroxyéthyle protégé, $R^2$ et $R^3$ sont, indépendamment, de l'hydrogène et $R^4$ est un groupe carboxy, (alkoxy en $C_1$ à $C_6$)carbonyle, alcanoyle en $C_1$ à $C_6$, arylcarbonyle, arylcarbonyloxy ou alcanoyloxy en $C_1$ à $C_6$.

**4.** Procédé suivant l'une quelconque des revendications 1 à 3, dans lequel l'atome d'azote du noyau d'azétidinone est substitué par un groupe de formule II :

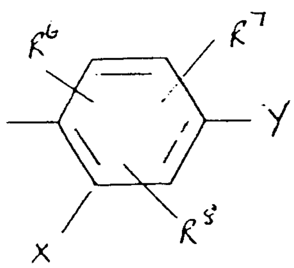

dans laquelle l'un de X et Y est un groupe $-OR^5$ dans lequel $R^5$ est l'hydrogène, un groupe protégeant la fonction hydroxy, un groupe alkyle en $C_1$ à $C_6$ facultativement substitué, phényle facultativement substitué ou alcényle en $C_2$ à $C_6$, l'autre de X et Y est un groupe $R^9$, et $R^6$ à $R^9$ sont choisis entre l'hydrogène, un groupe hydroxy, hydroxy protégé, alkyle en $C_1$ à $C_6$ facultativement substitué, alkoxy en $C_1$ à $C_6$ facultativement substitué, phénoxy facultativement substitué, alcényloxy en $C_2$ à $C_6$, halogéno, aryle et hétéroaryle,

ou est substitué par un groupe de formule III :

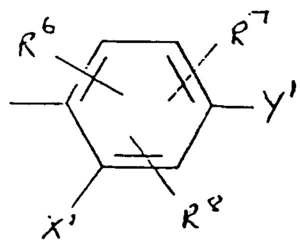

dans laquelle l'un de X' et Y' est un groupe $-NR^{10}R^{11}$ dans lequel $R^{10}$ et $R^{11}$ représentent, indépendamment, de l'hydrogène, un groupe alkyle en $C_1$ à $C_6$, par exemple méthyle, un groupe aryle, par exemple phényle ou un groupe acyle, par exemple alcanoyle en $C_1$ à $C_6$ tel qu'acétyle, l'autre de X' et Y' étant un groupe $R^9$, et $R^6$ à $R^9$ sont tels que définis ci-dessus.

**5.** Procédé suivant la revendication 1, dans lequel l'azétidinone est la 4-benzoyl-3-(1-hydroxyéthyl)-1-(p-méthoxyphényl)azétidine-2-one ou un dérivé à groupe hydroxy protégé de ce composé.

**6.** Procédé suivant l'une quelconque des revendications 1 à 5, dans lequel le milieu liquide comprend jusqu'à environ 50 % en volume/volume d'eau et un solvant organique principalement inerte, l'électrode de travail est principalement constituée de platine, de carbone, d'oxyde d'argent, d'oxyde de manganèse, d'oxyde de fer, d'or, de ruthénium, d'iridium ou de rhodium et le procédé est mis en oeuvre à une température comprise dans la plage d'environ $-5\,^\circ C$ à environ $+85\,^\circ C$.

**7.** Procédé suivant l'une quelconque des revendications 1 à 6, dans lequel l'électrode de travail est le platine ou le titane platiné.

**8.** Procédé suivant l'une quelconque des revendications 1 à 7, dans lequel le potentiel de l'électrode de travail se situe dans la plage de 1,2 à 1,8 volt par rapport à l'électrode de référence au calomel.

**9.** Procédé suivant l'une quelconque des revendications 1 à 8, dans lequel le pH est maintenu dans la plage de 4 à 7.